(19) Europäisches Patentamt
European Patent Office
Office européen des brevets

(11) **EP 4 382 146 A1**

(12) **EUROPEAN PATENT APPLICATION**
published in accordance with Art. 153(4) EPC

(43) Date of publication:
**12.06.2024 Bulletin 2024/24**

(21) Application number: **22878599.4**

(22) Date of filing: **06.10.2022**

(51) International Patent Classification (IPC):
*A61M 1/16* (2006.01)   *G01N 21/27* (2006.01)
*G01N 21/359* (2014.01)   *A61B 5/1455* (2006.01)

(52) Cooperative Patent Classification (CPC):
**A61B 5/1455; A61M 1/16; A61M 60/113;
A61M 60/279; A61M 60/37; A61M 60/443;
G01N 21/27; G01N 21/359**

(86) International application number:
**PCT/JP2022/037514**

(87) International publication number:
**WO 2023/058732 (13.04.2023 Gazette 2023/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.10.2021   JP 2021166517**

(71) Applicant: **Nikkiso Company Limited
Tokyo 150-6022 (JP)**

(72) Inventors:
• **YOSHINAGA, Kazuki
  Makinohara-shi, Shizuoka 421-0496 (JP)**

• **NAKAGAWA, Arata
  Makinohara-shi, Shizuoka 421-0496 (JP)**
• **MURAKAMI, Tomoya
  Tokyo 150-6022 (JP)**
• **HAMADA, Kimihiko
  Makinohara-shi, Shizuoka 421-0496 (JP)**
• **MATSUDA, Ayumi
  Makinohara-shi, Shizuoka 421-0496 (JP)**

(74) Representative: **von Hirschhausen, Helge
Grosse - Schumacher -
Knauer - von Hirschhausen
Patent- und Rechtsanwälte
Nymphenburger Straße 14
80335 München (DE)**

(54) **BLOOD PURIFICATION DEVICE**

(57)     To provide a blood purification apparatus that is capable of accurately acquiring the oxygen saturation regardless of variations in the blood concentration. A blood purification apparatus includes a first light-applying unit (8), a second light-applying unit (9), a third light-applying unit (10), a light-receiving unit (11), and an error-absorbing unit (12) configured to acquire oxygen saturation from a ratio (R/IR) between a first received-light intensity (R_660) and a second received-light intensity (IR_880) and to absorb any error in the oxygen saturation with reference to a third received-light intensity (IR_810) detected by the light-receiving unit (11). The error in the oxygen saturation occurs with a change in the blood concentration (hematocrit value) .

EP 4 382 146 A1

[ Fig. 1 ]

## Description

Technical Field

The present invention relates to a blood purification apparatus configured to purify a patient's blood by causing the blood to extracorporeally circulate through a blood purifier and a blood circuit.

Background Art

A hemodialysis apparatus for giving dialysis treatment typically includes a blood circuit including an arterial blood circuit and a venous blood circuit for causing a patient's blood to extracorporeally circulate, a dialyzer connected to the arterial blood circuit and to the venous blood circuit and serving as a blood purifier for purifying the blood that is under extracorporeal circulation, and an ultrafiltration pump capable of performing ultrafiltration in which excess water is removed from the blood flowing in the dialyzer. Thus, blood purification treatment is achieved while the blood extracorporeally circulating through the blood circuit is ultrafiltered.

In known arts, as disclosed by PTL 1 for example, a blood purification apparatus has been proposed that is capable of measuring the oxygen saturation of a patient's blood during blood purification treatment. In the known blood purification apparatus, a light-emitting unit emits first light having a wavelength that tends to be absorbed by oxyhemoglobin in the blood and second light having a wavelength that tends to be absorbed by reduced hemoglobin in the blood, and a light-receiving unit receives the reflections of the first light and the second light, whereby received-light intensities are detected. Thus, the oxygen saturation of the blood is measurable.

Citation List

Patent Literature

PTL 1: Japanese Unexamined Patent Application Publication No. 2012-40058

Summary of Invention

Technical Problem

In the above known blood purification apparatus, however, during the measurement of the oxygen saturation of the blood, the voltage of the received light to be detected by the light-receiving unit may change with changes in the patient's blood concentration, such as the hematocrit value. Consequently, the oxygen saturation acquired may have an error.

The present invention has been conceived in view of the above circumstances and is to provide a blood purification apparatus that is capable of accurately acquiring the oxygen saturation regardless of variations in the blood concentration.

Solution to Problem

A blood purification apparatus according to an embodiment of the present invention is a blood purification apparatus configured to purify a patient's blood by causing the blood to extracorporeally circulate through a blood purifier and a blood circuit. The apparatus includes a first light-applying unit configured to apply red light to the blood flowing in the blood circuit; a second light-applying unit configured to apply near infrared light to the blood flowing in the blood circuit; a third light-applying unit configured to apply detection light to the blood flowing in the blood circuit, the detection light having a different wavelength from the red light and the near infrared light to be emitted from the first light-applying unit and the second light-applying unit, the detection light enabling detection of blood concentration regardless of an oxygen saturation of the blood; a light-receiving unit configured to detect a first received-light intensity acquired by receiving the red light emitted from the first light-applying unit and reflected by the blood or transmitted through the blood, a second received-light intensity acquired by receiving the near infrared light emitted from the second light-applying unit and reflected by the blood or transmitted through the blood, and a third received-light intensity acquired by receiving the detection light emitted from the third light-applying unit and reflected by the blood or transmitted through the blood; and an error-absorbing unit configured to acquire the oxygen saturation from a ratio between the first received-light intensity and the second received-light intensity and to absorb any error in the oxygen saturation with reference to the third received-light intensity detected by the light-receiving unit, the error in the oxygen saturation occurring with a change in the blood concentration.

Advantageous Effects of Invention

According to the present invention, any error in the oxygen saturation that may occur with a change in the blood concentration is absorbed with reference to the third received-light intensity detected by the light-receiving unit. Such a configuration enables the accurate acquisition of the oxygen saturation regardless of variations in blood concentration.

Brief Description of Drawings

[Fig. 1] Fig. 1 schematically illustrates a blood purification apparatus according to a first embodiment of the present invention.
[Fig. 2] Fig. 2 is a front view of a hematocrit sensor included in the blood purification apparatus.
[Fig. 3] Fig. 3 illustrates a section taken along line III-III given in Fig. 2.
[Fig. 4] Fig. 4 illustrates a section taken along line IV-IV given in Fig. 2.
[Fig. 5] Fig. 5 is a graph illustrating the results of an experiment conducted for acquiring a correction factor to be used in correcting a calibration curve in the blood purification apparatus.
[Fig. 6] Fig. 6 is a graph illustrating a calibration curve to be applied in the blood purification apparatus.
[Fig. 7] Fig. 7 is a flow chart illustrating a process of acquiring oxygen saturation in the blood purification apparatus.
[Fig. 8] Fig. 8 schematically illustrates a blood purification apparatus according to a second embodiment of the present invention.
[Fig. 9] Fig. 9 is a flow chart illustrating a process of acquiring oxygen saturation in the blood purification apparatus.
[Fig. 10] Fig. 10 schematically illustrates a blood purification apparatus according to a third embodiment of the present invention.
[Fig. 11] Fig. 11 is a graph illustrating calibration curves to be applied in the blood purification apparatus.
[Fig. 12] Fig. 12 is a flow chart illustrating a process of acquiring oxygen saturation in the blood purification apparatus.
[Fig. 13] Fig. 13 schematically illustrates a blood purification apparatus according to a fourth embodiment of the present invention.
[Fig. 14] Fig. 14 is a flow chart illustrating a process of acquiring oxygen saturation in the blood purification apparatus.

Description of Embodiments

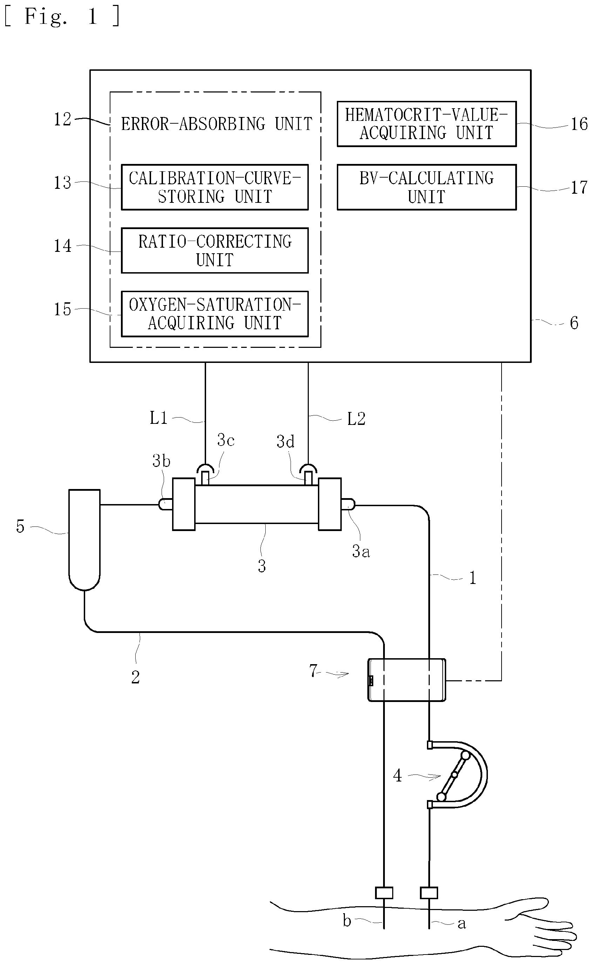

Embodiments of the present invention will now be described specifically with reference to the drawings.
A blood purification apparatus according to a first embodiment is a dialysis apparatus for giving dialysis treatment and includes, as illustrated in Fig. 1, a blood circuit, which includes an arterial blood circuit 1 and a venous blood circuit 2; a dialyzer 3 (a blood purifier), which is interposed between the arterial blood circuit 1 and the venous blood circuit 2 and is configured to purify blood that flows through the blood circuit; a blood pump 4; an air-trap chamber 5, which is provided to the venous blood circuit 2; a dialysis device 6, which is configured to supply dialysate to the dialyzer 3 and discharge drain liquid from the dialyzer 3; and a blood-index detector 7.
The arterial blood circuit 1 is provided at the distal end thereof with a connector, to which an arterial puncture needle a is connectable. The blood pump 4 is of a peristaltic type and is provided at a halfway position of the arterial blood circuit 1. The venous blood circuit 2 is provided at the distal end thereof with a connector, to which a venous puncture needle b is connectable. The air-trap chamber 5 is provided at a halfway position of the venous blood circuit 2. The air-trap chamber 5 has an air layer formed therein so that bubbles in fluid are trappable therein.
The blood pump 4 is a peristaltic pump provided to the arterial blood circuit 1 and is capable of causing fluid in the blood circuit to flow in an operating direction. Specifically, the arterial blood circuit 1 is provided with a squeezable tube (as a portion of the arterial blood circuit 1) that is softer and has a greater diameter than other flexible tubes forming the arterial blood circuit 1. The blood pump 4 includes rollers for squeezing the squeezable tube in the direction of fluid delivery. When the blood pump 4 is activated, the rollers rotate and squeeze the squeezable tube in the direction of fluid delivery, whereby the fluid inside the tube is caused to flow in the operating direction (the direction of rotation of the rollers).
When the blood pump 4 is activated while a patient is punctured with the arterial puncture needle a and the venous puncture needle b, the patient's blood flows through the arterial blood circuit 1 and reaches the dialyzer 3, where the blood is purified. Then, the blood flows through the venous blood circuit 2 while being subjected to bubble removal in the air-trap chamber 5, and returns into the patient's body. That is, the patient's blood is purified by the dialyzer 3 while the blood is caused to extracorporeally circulate through the blood circuit from the distal end of the arterial blood circuit 1 to the distal end of the venous blood circuit 2.
The dialyzer 3 includes a housing. The housing has a blood introduction port 3a, a blood delivery port 3b, a dialysate introduction port 3c, and a dialysate delivery port 3d. The blood introduction port 3a is connected to the arterial blood circuit 1. The blood delivery port 3b is connected to the venous blood circuit 2. The dialysate introduction port 3c and the dialysate delivery port 3d are connected to a dialysate introduction line L1 and a dialysate discharge line L2, respectively, which extend from the dialysis device 6.

The dialyzer 3 houses a plurality of hollow fiber membranes. The insides of the hollow fiber membranes serve as blood flow routes. The spaces between the inner peripheral surface of the housing of the dialyzer 3 and the outer peripheral surfaces of the hollow fiber membranes serve as dialysate flow routes. The hollow fiber membranes each have a number of microscopic holes (pores) extending therethrough from the outer peripheral surface to the inner peripheral surface. Impurities and the like contained in the blood are allowed to permeate through the hollow fiber membranes into the dialysate, whereby the blood is purifiable.

On the other hand, the dialysis device 6 includes a fluid delivery device, such as a duplex pump, provided astride the dialysate introduction line L1 and the dialysate discharge line L2. A bypass line that bypasses the fluid delivery device is provided with an ultrafiltration pump configured to remove water from the patient's blood flowing in the dialyzer 3. The dialysate introduction line L1 is connected at one end thereof to the dialyzer 3 (the dialysate introduction port 3c) and at the other end thereof to a dialysate supply device (not illustrated) configured to prepare a dialysate at a predetermined concentration. The dialysate discharge line L2 is connected at one end thereof to the dialyzer 3 (the dialysate delivery port 3d) and at the other end thereof to a draining device, which is not illustrated. The dialysate supplied from the dialysate supply device flows through the dialysate introduction line L1 into the dialyzer 3, and then flows through the dialysate discharge line L2 into the draining device.

The air-trap chamber 5 is provided with a monitor tube, to which a pressure sensor is connected so that the fluid pressure (venous pressure) in the air-trap chamber 5 is measurable. The air-trap chamber 5 is further provided with an overflow line, which extends from an upper part (on the air-layer side) of the air-trap chamber 5. The overflow line is provided with an electromagnetic valve at a halfway position thereof. When the electromagnetic valve is opened, the fluid (a priming solution or the like) flowing in the blood circuit is allowed to overflow through the overflow line.

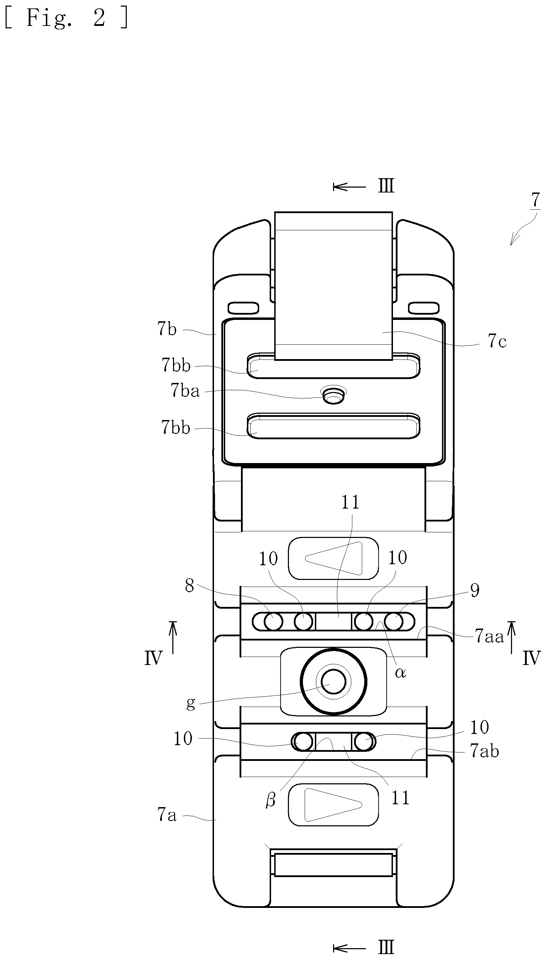

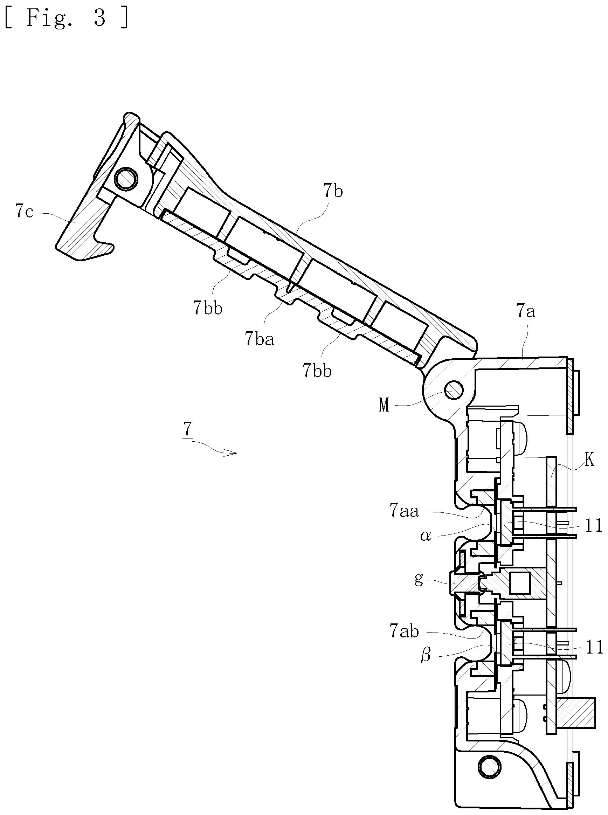

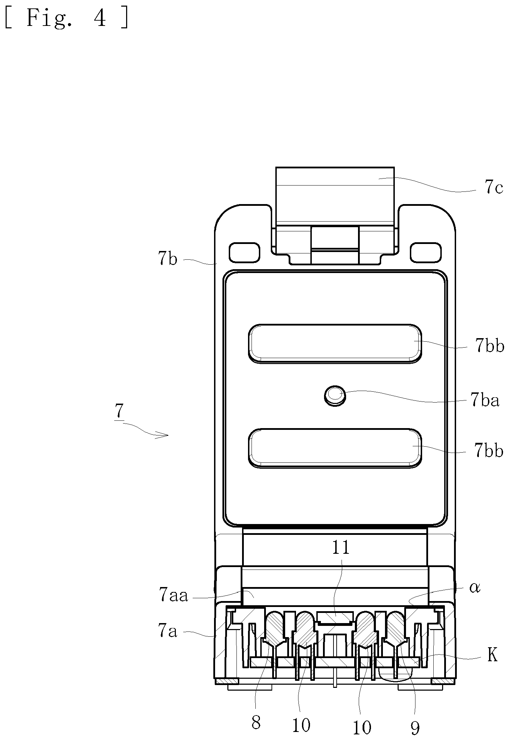

A blood-index detector 7 is attached to predetermined positions of the arterial blood circuit 1 and the venous blood circuit 2. The blood-index detector 7 is configured to apply light to the blood flowing in the arterial blood circuit 1 and the venous blood circuit 2 and to receive the reflection of the light, thereby acquiring the voltage of the light received. With reference to the received-light voltage, the blood-index detector 7 is capable of calculating or acquiring oxygen saturation, the hematocrit value, and the circulating-blood-volume rate of change ($\Delta$BV). As illustrated in Figs. 2 to 4, the blood-index detector 7 includes a first light-applying unit 8, a second light-applying unit 9, and third light-applying units 10, which all include light-emitting devices (LEDs); and light-receiving units 11, which include light-receiving devices (photodiodes).

The blood-index detector 7 according to the present embodiment further includes a body 7a, a lid 7b, and a detection switch g. The body 7a has fitting grooves 7aa and 7ab, into which respective portions (flexible tubes) of the blood circuit are fittable. The lid 7b is openable and closable on the body 7a and is capable of nipping, when closed, the portions of the blood circuit fitted in the fitting grooves 7aa and 7ab. The detection switch g is configured to detect whether the lid 7b is opened or closed. For example, one of the flexible tubes forming the arterial blood circuit 1 is fittable into the fitting groove 7aa, while one of the flexible tubes forming the venous blood circuit 2 is fittable into the fitting groove 7ab. In the present embodiment, the flexible tubes of the blood circuit are press-fitted into the respective fitting grooves 7aa and 7ab, thereby being firmly secured for improved detection accuracy. Alternatively, for example, the flexible tubes may be loosely fitted or may be freed.

The lid 7b is swingably attached to the body 7a with the aid of a swing shaft M and is openable and closable by being swung about the swing shaft M. The lid 7b includes a locking part 7c. When the lid 7b is closed, the locking part 7c is lockable onto the body 7a. With the locking of the locking part 7c, the flexible tubes are assuredly kept nipped. When the lid 7b is closed with the flexible tube of the arterial blood circuit 1 and the flexible tube of the venous blood circuit 2 fitted in the respective fitting grooves 7aa and 7ab, the flexible tubes are nippable between the body 7a and the lid 7b from above and below.

The detection switch g is a microswitch provided on the body 7a between the fitting groove 7aa and the fitting groove 7ab. When the lid 7b is swung about the swing shaft M and is brought close to the body 7a, the detection switch g is pushed to be turned on by a protrusion 7ba, which is provided on the lid 7b. With the detection switch g turned on or off, whether the lid 7b is open or closed is detectable.

The fitting grooves 7aa and 7ab provided in the body 7a have respective slits $\alpha$ and $\beta$. The slits $\alpha$ and $\beta$ are cuts made at the bottoms of the fitting grooves 7aa and 7ab and each extend over a predetermined length in the elongated direction of the fitting grooves 7aa and 7ab. The body 7a includes thereinside a printed circuit board K. The first light-applying unit 8, the second light-applying unit 9, the third light-applying units 10, and the light-receiving unit 11 are positioned on the inner side relative to the slit $\alpha$. The third light-applying units 10 and the light-receiving unit 11 are positioned on the inner side relative to the slit $\beta$. With the slits $\alpha$ and $\beta$ provided as above, disturbing light outside the body 7a is less likely to reach the light-receiving units 11, and light beams emitted from the first light-applying unit 8, the second light-applying unit 9, and the third light-applying units 10 exhibit improved linearity. Note that the slits $\alpha$ and $\beta$

may be omitted.

In the above configuration (a reflection-sensor configuration), the light beams emitted from the first light-applying unit 8, the second light-applying unit 9, and the third light-applying units 10 are allowed to travel through the slit $\alpha$ and to be reflected by the blood flowing in the arterial blood circuit 1, and the light beams emitted from the third light-applying units 10 are allowed to travel through the slit $\beta$ and to be reflected by the blood flowing in the venous blood circuit 2. The reflected light beams are receivable by the light-receiving units 11. While the blood-index detector 7 according to the present embodiment is a reflection sensor, the blood-index detector 7 may alternatively be a transmission sensor capable of applying light to the blood flowing in the blood circuit and receiving the light transmitted through the blood.

The fitting grooves 7aa and 7ab each extend from one end part (the right end part in Fig. 2) of the body 7a to the other end part (the left end part in Fig. 2) of the body 7a. The light-receiving units 11 are positioned at the centers of the respective fitting grooves 7aa and 7ab. The first light-applying unit 8, the second light-applying unit 9, the third light-applying units 10, and the light-receiving units 11 are linearly arrayed on the single printed circuit board K provided inside the body 7a. The light-receiving units 11 are positioned between the first light-applying unit 8, the second light-applying unit 9, and the third light-applying units 10 (specifically, between respective pairs of the third light-applying units 10). More specifically, in the slit $\alpha$, one pair of third light-applying units 10 are positioned on two respective sides of a light-receiving unit 11, and the first light-applying unit 8 and the second light-applying unit 9 are positioned on the respective outer sides of the third light-applying units 10. In such an arrangement in which the light-receiving unit 11 is positioned between the light-emitting units (the first light-applying unit 8, the second light-applying unit 9, and the third light-applying units 10), the influence of disturbing light can be minimized.

The lid 7b is further provided with light-absorbing parts 7bb at positions that face the first light-applying unit 8, the second light-applying unit 9, the third light-applying units 10, and the light-receiving units 11. The light-absorbing parts 7bb absorb the light beams emitted from the first light-applying unit 8, the second light-applying unit 9, and the third light-applying units 10. The light-absorbing parts 7bb may each be any of the following, for example: a part that has a dark color such as a black color and thus absorbs light, and a part that is made of a light-absorbing material. Hence, the light beams emitted from the first light-applying unit 8, the second light-applying unit 9, and the third light-applying units 10 and reflected by the blood are received by the light-receiving units 11, whereas light transmitted through the blood is absorbed by the light-absorbing parts 7bb so as not to reach the light-receiving units 11.

The first light-applying unit 8 includes an LED (a red-light LED) capable of applying red light (red light having a wavelength of 660 nm $\pm$ 20 nm) to the blood flowing in the blood circuit. The second light-applying unit 9 includes an LED (a near-infrared-light LED) capable of applying near infrared light (near infrared light having a wavelength of 880 nm (+ 15 nm or - 5nm)) to the blood flowing in the blood circuit. Specifically, the red light to be emitted from the first light-applying unit 8 has a wavelength (a wavelength of 660 nm) characterized by an absorbance that is higher for reduced hemoglobin (Hb) contained in the blood than for oxyhemoglobin (HbO2) contained in the blood. The near infrared light to be emitted from the second light-applying unit 9 has a wavelength (a wavelength of 880 nm) characterized by an absorbance that is higher for oxyhemoglobin contained in the blood than for reduced hemoglobin contained in the blood.

The third light-applying units 10 each include an LED (a near-infrared-light LED) capable of applying detection light (an isosbestic-point wavelength) to the blood flowing in the blood circuit. The detection light has a different wavelength (near infrared light having a wavelength of 810 nm $\pm$ 10 nm) from the red light and the near infrared light to be emitted from the first light-applying unit 8 and the second light-applying unit 9. The detection light enables the detection of the blood concentration (hematocrit value) regardless of the oxygen saturation of the blood. In other words, the detection light to be emitted from the third light-applying units 10 has a wavelength (a wavelength of 810 nm $\pm$ 10 nm) characterized by an absorbance that is substantially equal between oxyhemoglobin (HbO2) contained in the blood and reduced hemoglobin (Hb) contained in the blood.

The light-receiving units 11 each include a photodiode provided on the printed circuit board K together with the first light-applying unit 8, the second light-applying unit 9, and the third light-applying units 10. The light-receiving units 11 are capable of detecting a first received-light intensity (R_660) acquired by receiving the red light emitted from the first light-applying unit 8 and reflected by the blood (or transmitted through the blood), a second received-light intensity (IR_880) acquired by receiving the near infrared light emitted from the second light-applying unit 9 and reflected by the blood (or transmitted through the blood), and a third received-light intensity (IR_810) acquired by receiving the detection light emitted from the third light-applying units 10 and reflected by the blood (or transmitted through the blood). If the light-receiving units 11 are configured to receive light transmitted through the blood, the light-absorbing parts 7bb of the lid 7b need to be provided with sensors configured to receive transmitted light. Note that the first received-light intensity (R_660), the second received-light intensity (IR_880), and the third received-light intensity (IR_810) are to be detected as voltages (received-light voltages).

The dialysis device 6 according to the present embodiment includes an error-absorbing unit 12, a hematocrit-value-acquiring unit 16, and a BV-calculating unit 17. The error-absorbing unit 12 includes a calibration-curve-storing unit 13, a ratio-correcting unit 14, and an oxygen-saturation-acquiring unit 15. The error-absorbing unit 12 includes, for example, a microcomputer and a storage that are electrically connected to the blood-index detector 7. The error-absorbing unit 12 is configured to acquire oxygen saturation ($SO_2$(ABL)%) from the ratio (R/IR) between the first received-light intensity (R_660) and the second received-light intensity (IR_880) and to absorb any error in the oxygen saturation with reference to the third received-light intensity (IR_810) detected by the light-receiving units 11. The error in the oxygen saturation may occur with a change in the blood concentration (hematocrit value).

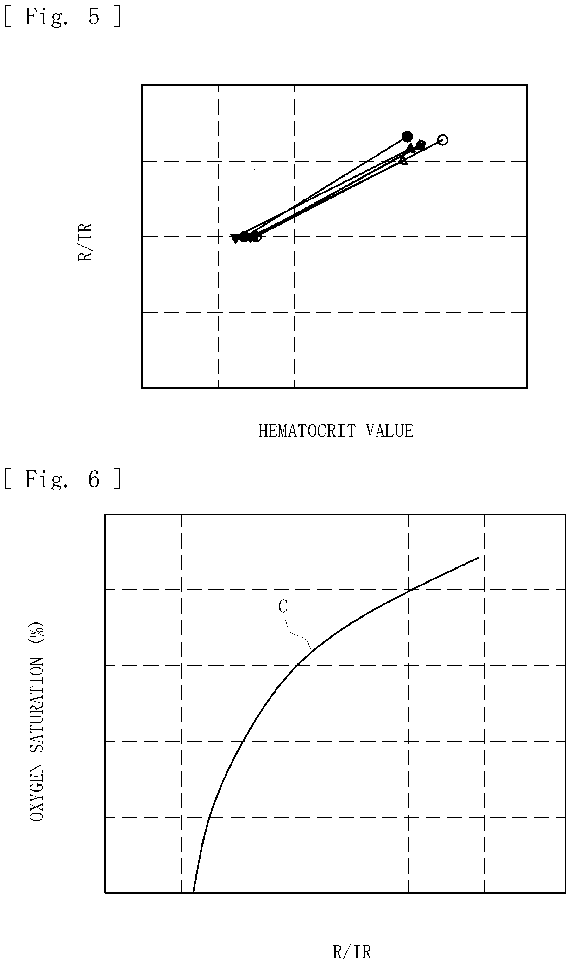

The calibration-curve-storing unit 13 is configured to store in advance a calibration curve C, which is used in obtaining the oxygen saturation of the blood from the ratio (R/IR) between the first received-light intensity (R_660) and the second received-light intensity (IR_880). As illustrated in Fig. 6, the calibration curve C is a curve (cubic curve) defined by a horizontal axis representing the ratio (R/IR) between the first received-light intensity (R_660) and the second received-light intensity (IR_880) and a vertical axis representing oxygen saturation. The calibration curve C is generated from values acquired in an experiment or the like conducted in advance as to be described below.

The ratio-correcting unit 14 is configured to correct, with reference to the third received-light intensity (IR_810) detected by the light-receiving units 11, the ratio (R/IR) between the first received-light intensity (R_660) and the second received-light intensity (IR_880) detected by the light-receiving units 11. The ratio-correcting unit 14 is capable of performing the correction by using a correction factor acquired in an experiment or the like conducted in advance as to be described below.

In the experiment or the like to be conducted in advance, for example, the detection light emitted from the third light-applying units 10 is applied to bloods having different blood concentrations (hematocrit values) (in the present experiment, bloods having respective hematocrit values (Ht) of 20% and 40%), whereby the blood concentrations (hematocrit values) are acquired. Furthermore, the red light and the near infrared light emitted from the first light-applying unit 8 and the second light-applying unit 9 are applied, whereby the first received-light intensity (R_660) and the second received-light intensity (IR_880) are acquired. Thus, a plurality of lines illustrated in Fig. 5 are obtained.

Then, a line (approximate line y = ax + b) representing the average of the plurality of lines is calculated. Here, letting the received-light-voltage ratio (R/IR) for the hematocrit value (Ht) of 20% be XHt20 and the hematocrit value calculated therefor be (Ht20) and further letting the received-light-voltage ratio (R/IR) for the hematocrit value (Ht) of 40% be XHt40 and the hematocrit value calculated therefor be (Ht40), a and b to be used in expressing the approximate line are calculable through the following mathematical expressions:

$$a = ((XHt40/XHt20) - 1)/(Ht40 - Ht20) = 0.0060$$

$$b = XHt40/XHt20 - a×Ht40 = 0.8967$$

Accordingly, the correction factor, K4, based on the hematocrit value (Ht) calculated from the third received-light intensity (IR_810) and the received-light-voltage ratio, Xa, obtained after the correction are expressed as follows, with the received-light-voltage ratio before the correction being defined as X; and the hematocrit value calculated from the third received-light intensity (IR_810) being defined as *Ht. Note that *Ht needs to be subjected to zero and span adjustment by using an adjuster.

$$K4 = 0.0060×*Ht + 0.8967$$

$$Xa = X/K4$$

With reference to the relationship between the corrected received-light-voltage ratio Xa and the oxygen saturation, the calibration curve C to be stored in the calibration-curve-storing unit 13 is calculable as illustrated in Fig. 6, and the correction factor K4 to be used in correcting the received-light-voltage ratio (R/IR) is acquirable by the ratio-correcting unit 14. That is, with reference to the third received-light intensity (IR_810) acquired by applying from the third light-applying unit 10 the wavelength (the wavelength of 810 nm) having an absorbance that is equal between oxyhemoglobin (HbO2) and reduced hemoglobin (Hb), the hematocrit value is acquired under a reduced influence of oxygen saturation. Using the hematocrit value thus acquired, the correction factor K4 and the calibration curve C are calculable.

The oxygen-saturation-acquiring unit 15 is configured to acquire, with reference to the calibration curve C stored in the calibration-curve-storing unit 13, the oxygen saturation of the blood from the ratio (R/IR), corrected with the correction factor K4 by the ratio-correcting unit 14, between the first received-light intensity and the second received-light intensity. The oxygen saturation thus acquired by the oxygen-saturation-acquiring unit 15 is accurate because even if the hematocrit value changes during blood purification treatment, an error in the oxygen saturation that results from such a change in the hematocrit value is absorbed.

The hematocrit-value-acquiring unit 16 is configured to acquire the hematocrit value (Ht) with reference to the third received-light intensity (IR_810) detected by the light-receiving units 11. Blood components such as red blood cells and plasma have respective inherent absorption characteristics. Using such characteristics, the hematocrit value (Ht) is calculable by electrooptically quantifying red blood cells necessary for the measurement of the hematocrit value. Specifically, when the near infrared light emitted from the third light-applying units 10 is reflected by the blood, the near infrared light undergoes the influence of absorption and scattering before being received by the light-receiving units 11. The rate of absorptive scattering of the light is analyzed from the intensity of the received light, whereby the hematocrit value (Ht) is acquired.

The BV-calculating unit 17 is configured to calculate the circulating-blood-volume rate of change (ΔBV) with reference to the hematocrit value (Ht) acquired by the hematocrit-value-acquiring unit 16. Specifically, the BV-calculating unit 17 is configured to calculate the circulating-blood-volume rate of change (ΔBV) by using the hematocrit value (Ht) acquired by the hematocrit-value-acquiring unit 16 and through the following mathematical expression: $\Delta BV (\%) = (Ht_0/Ht_t - 1) \times 100$ (where $Ht_0$ denotes the hematocrit value at the beginning of the treatment, and $Ht_t$ denotes the hematocrit value at the time of measurement). The circulating-blood-volume rate of change (ΔBV) thus calculated is taken as a reference for the ultrafiltration rate and the ultrafiltration volume. The operation of the ultrafiltration pump is controlled with reference to the hematocrit value acquired as above or the circulating-blood-volume rate of change ΔBV calculated from the hematocrit value, so that the ultrafiltration rate and the ultrafiltration volume can be made to match the patient's condition.

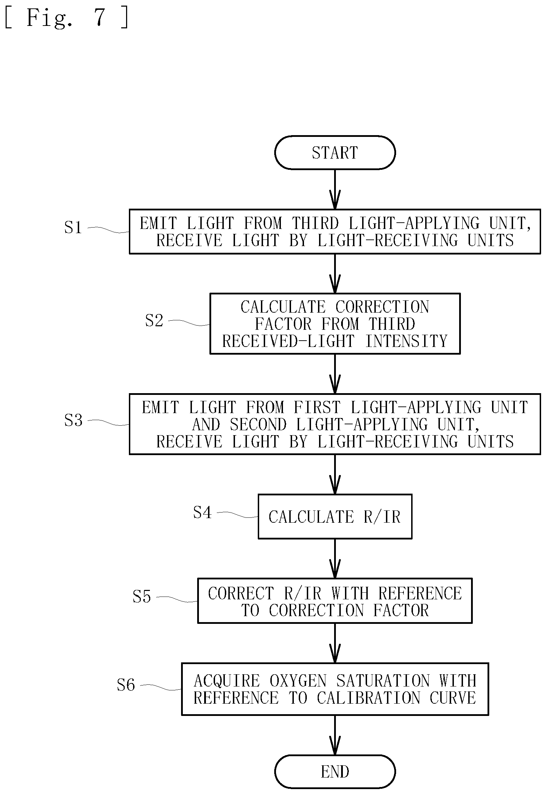

Now, the process of calculating the oxygen saturation according to the present embodiment will be described with reference to the flow chart illustrated in Fig. 7.

A calibration curve C is stored in advance in the calibration-curve-storing unit 13, and the patient's blood is caused to extracorporeally circulate through the blood circuit. Then, the third light-applying units 10 emit the detection light, and the light-receiving units 11 receive the reflection of the detection light to acquire the third received-light intensity (IR_810) (S1). With reference to the third received-light intensity (IR_810) thus acquired, the correction factor K4 is calculated (S2).

Subsequently, the first light-applying unit 8 and the second light-applying unit 9 emit the red light and the near infrared light, and the light-receiving units 11 acquire the first received-light intensity (R_660) and the second received-light intensity (IR_880) (S3). Then, the ratio (R/IR) between the first received-light intensity (R_660) and the second received-light intensity (IR_880) is calculated (S4). With reference to the correction factor K4 obtained in S2, the ratio-correcting unit 14 corrects the ratio (R/IR) (S5). With reference to the ratio (R/IR) thus corrected and the calibration curve C stored in the calibration-curve-storing unit 13, the oxygen-saturation-acquiring unit 15 acquires the oxygen saturation (S6).

According to the present embodiment, the error-absorbing unit 12 includes the calibration-curve-storing unit 13 configured to store in advance a calibration curve C to be used in obtaining the oxygen saturation of the blood from the ratio (R/IR) between the first received-light intensity (R_660) and the second received-light intensity (IR_880); the ratio-correcting unit 14 configured to correct, with reference to the third received-light intensity (IR_810) detected by the light-receiving units 11, the ratio (R/IR) between the first received-light intensity (R_660) and the second received-light intensity (IR_880) detected by the light-receiving units 11; and the oxygen-saturation-acquiring unit 15 configured to acquire the oxygen saturation of the blood from the ratio (R/IR), corrected by the ratio-correcting unit 14, between the first received-light intensity (R_660) and the second received-light intensity (IR_880) with reference to the calibration curve C stored in the calibration-curve-storing unit 13. Therefore, the oxygen saturation can be acquired with no changes in the calibration curve C stored in the calibration-curve-storing unit 13.

Now, a blood purification apparatus according to a second embodiment of the present invention will be described. Elements that are the same as in the above embodiment are denoted by corresponding ones of the reference signs, and detailed description of such elements is omitted.

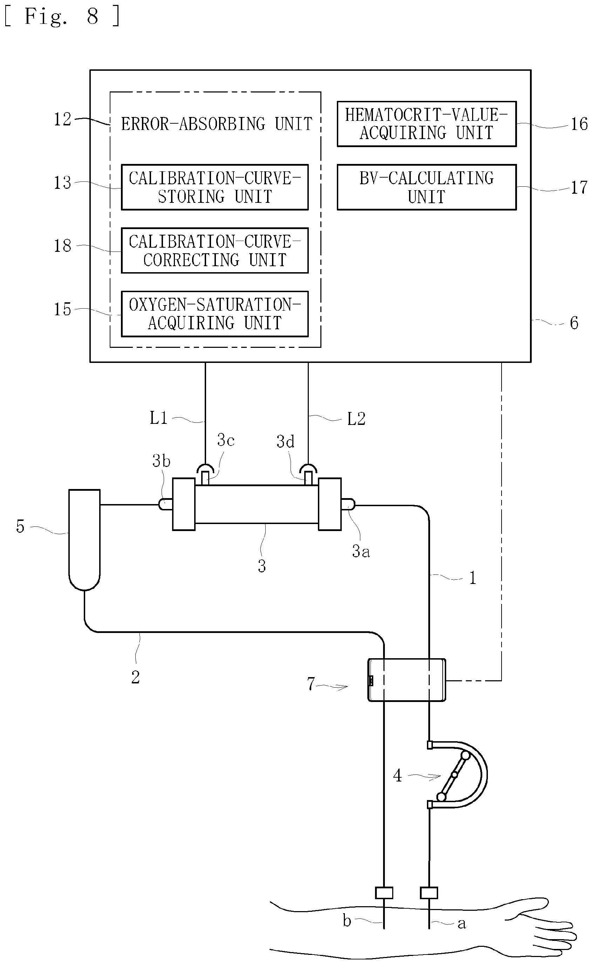

A blood purification apparatus according to the present embodiment is a dialysis apparatus for giving dialysis treatment and includes, as illustrated in Fig. 8, a blood circuit, which includes an arterial blood circuit 1 and a venous blood circuit 2; a dialyzer 3 (a blood purifier), which is interposed between the arterial blood circuit 1 and the venous blood circuit 2 and is configured to purify blood that flows through the blood circuit; a blood pump 4; an air-trap chamber 5, which is provided to the venous blood circuit 2; a dialysis device 6, which is configured to supply dialysate to the dialyzer 3 and discharge drain liquid from the dialyzer 3; and a blood-index detector 7.

The dialysis device 6 according to the present embodiment includes an error-absorbing unit 12, a hematocrit-value-acquiring unit 16, and a BV-calculating unit 17. The error-absorbing unit 12 includes a calibration-curve-storing unit 13, a calibration-curve-correcting unit 18, and an oxygen-saturation-acquiring unit 15. The error-absorbing unit 12 includes, for example, a microcomputer and a storage that are electrically connected to the blood-index detector 7. The error-absorbing unit 12 is configured to acquire oxygen saturation (SO₂(ABL)%) from the ratio (R/IR) between the first received-light intensity (R_660) and the second received-light intensity (IR_880) and to absorb any error in the oxygen saturation with reference to the third received-light intensity (IR_810) detected by the light-receiving units 11. The error in the oxygen saturation may occur with a change in the blood concentration (hematocrit value).

The calibration-curve-correcting unit 18 is configured to correct, with reference to the third received-light intensity (IR_810) detected by the light-receiving units 11, the calibration curve stored in the calibration-curve-storing unit 13. To summarize, the first embodiment employs a calibration curve C that does not change with the change in the hematocrit value. In contrast, the present embodiment employs a calibration curve that is corrected in real time in correspondence with the change in the hematocrit value.

The oxygen-saturation-acquiring unit 15 is configured to acquire, with reference to the calibration curve corrected by the calibration-curve-correcting unit 18, the oxygen saturation of the blood from the ratio (R/IR) between the first received-light intensity (R_660) and the second received-light intensity (IR_880) detected by the light-receiving units 11. To summarize, the first embodiment employs an oxygen-saturation-acquiring unit 15 that is configured to correct, with the correction factor K4, the ratio (R/IR) between the first received-light intensity and the second received-light intensity detected by the light-receiving units 11 and to calculate the oxygen saturation from the corrected ratio (R/IR) and the calibration curve C. In contrast, the present embodiment employs an oxygen-saturation-acquiring unit 15 that is configured to calculate the oxygen saturation from the ratio (R/IR) between the first received-light intensity and the second received-light intensity detected by the light-receiving units 11 and the calibration curve corrected in real time.

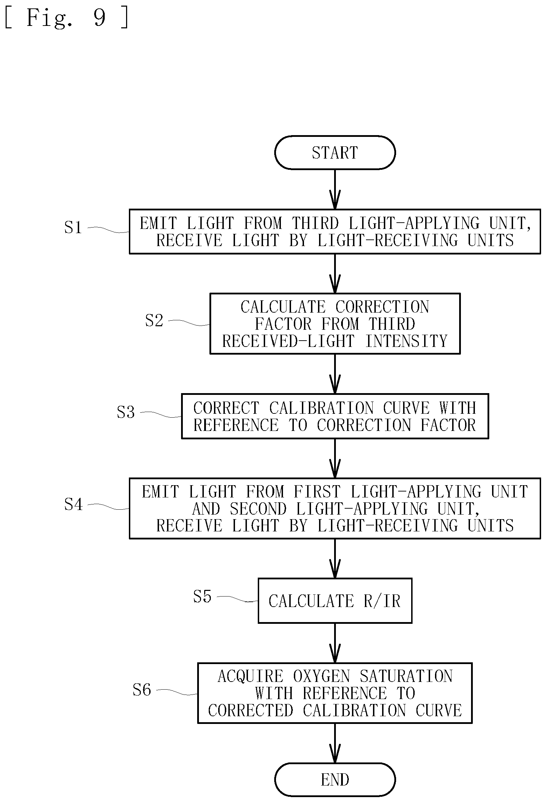

Now, the process of calculating the oxygen saturation according to the present embodiment will be described with reference to the flow chart illustrated in Fig. 9.

A calibration curve (an initial calibration curve) is stored in advance in the calibration-curve-storing unit 13, and the patient's blood is caused to extracorporeally circulate through the blood circuit. Then, the third light-applying units 10 emit the detection light, and the light-receiving units 11 receive the reflection of the detection light to acquire the third received-light intensity (IR_810) (S1). With reference to the third received-light intensity (IR_810) thus acquired, the correction factor is calculated (S2).

Subsequently, with reference to the correction factor calculated in S2, the calibration curve stored in the calibration-curve-storing unit 13 is corrected (S3). Furthermore, the first light-applying unit 8 and the second light-applying unit 9 emit the red light and the near infrared light, and the light-receiving units 11 acquire the first received-light intensity (R_660) and the second received-light intensity (IR_880) (S4). Then, the ratio (R/IR) between the first received-light intensity (R_660) and the second received-light intensity (IR_880) is calculated (S5). Subsequently, with reference to the ratio (R/IR) thus calculated and the calibration curve corrected in S3, the oxygen-saturation-acquiring unit 15 acquires the oxygen saturation (S6).

According to the present embodiment, the error-absorbing unit 12 includes the calibration-curve-storing unit 13 configured to store in advance a calibration curve to be used in obtaining the oxygen saturation of the blood from the ratio (R/IR) between the first received-light intensity (R_660) and the second received-light intensity (IR_880); the calibration-curve-correcting unit 18 configured to correct, with reference to the third received-light intensity (IR_810) detected by the light-receiving units 11, the calibration curve stored in the calibration-curve-storing unit 13; and the oxygen-saturation-acquiring unit 15 configured to acquire, with reference to the calibration curve corrected by the calibration-curve-correcting unit 18, the oxygen saturation of the blood from the ratio (R/IR) between the first received-light intensity (R_660) and the second received-light intensity (IR_880) detected by the light-receiving units 11. Therefore, the oxygen saturation can be acquired with no correction of the ratio (R/IR) between the first received-light intensity (R_660) and the second received-light intensity (IR_880) detected by the light-receiving units 11.

Now, a blood purification apparatus according to a third embodiment of the present invention will be described. Elements that are the same as in the above embodiments are denoted by corresponding ones of the reference signs, and detailed description of such elements is omitted.

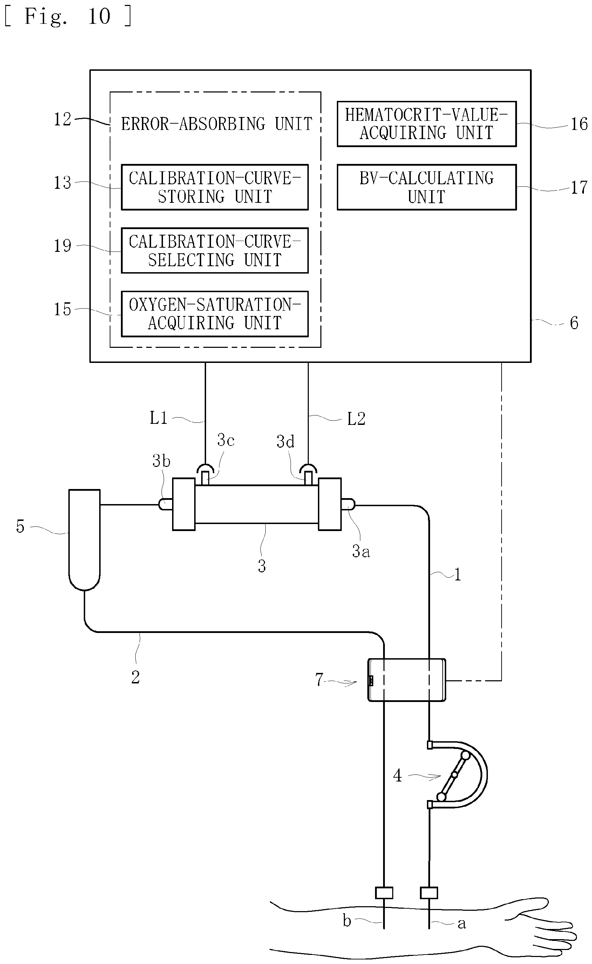

A blood purification apparatus according to the present embodiment is a dialysis apparatus for giving dialysis treatment and includes, as illustrated in Fig. 10, a blood circuit, which includes an arterial blood circuit 1 and a venous blood circuit 2; a dialyzer 3 (a blood purifier), which is interposed between the arterial blood circuit 1 and the venous blood circuit 2 and is configured to purify blood that flows through the blood circuit; a blood pump 4; an air-trap chamber 5, which is provided to the venous blood circuit 2; a dialysis device 6, which is configured to supply dialysate to the dialyzer 3 and discharge drain liquid from the dialyzer 3; and a blood-index detector 7.

The dialysis device 6 according to the present embodiment includes an error-absorbing unit 12, a hematocrit-value-acquiring unit 16, and a BV-calculating unit 17. The error-absorbing unit 12 includes a calibration-curve-storing unit 13, a calibration-curve-selecting unit 19, and an oxygen-saturation-acquiring unit 15. The error-absorbing unit 12 includes, for example, a microcomputer and a storage that are electrically connected to the blood-index detector 7. The error-absorbing unit 12 is configured to acquire oxygen saturation ($SO_2$(ABL)%) from the ratio (R/IR) between the first received-light intensity (R_660) and the second received-light intensity (IR_880) and to absorb any error in the oxygen saturation with reference to the third received-light intensity (IR_810) detected by the light-receiving units 11. The error in the oxygen saturation may occur with a change in the blood concentration (hematocrit value).

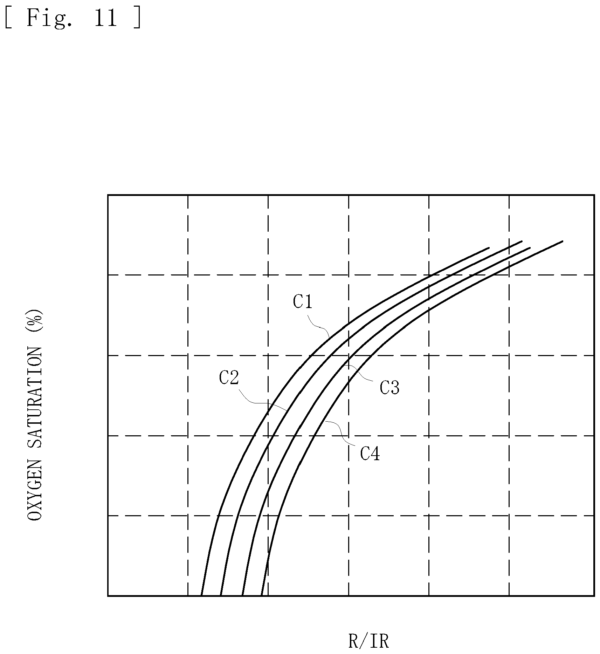

The calibration-curve-storing unit 13 is configured to store in advance a plurality of calibration curves (C1 to C4), which are illustrated in Fig. 11 and are to be used in obtaining the oxygen saturation of the blood from the ratio (R/IR) between the first received-light intensity (R_660) and the second received-light intensity (IR_880). The calibration curves (C1 to C4) are generated in correspondence with blood concentrations (hematocrit values). Note that an appropriate number of calibration curves are storable in advance in correspondence with blood concentrations.

The calibration-curve-selecting unit 19 is configured to select, with reference to the third received-light intensity (IR_810) detected by the light-receiving units 11, a particular one of the plurality of calibration curves (C1 to C4) stored in the calibration-curve-storing unit 13. To summarize, the first and second embodiments each employ a single calibration curve C. In contrast, the present embodiment employs a plurality of calibration curves that are prepared in advance in correspondence with hematocrit values.

The oxygen-saturation-acquiring unit 15 is configured to acquire, with reference to the calibration curve selected by the calibration-curve-selecting unit 19, the oxygen saturation of the blood from the ratio (R/IR) between the first received-light intensity (R_660) and the second received-light intensity (IR_880) detected by the light-receiving units 11. To summarize, the first and second embodiments each employ an oxygen-saturation-acquiring unit 15 that is configured to calculate the oxygen saturation with reference to the calibration curve corrected in advance with a correction factor or corrected in real time. In contrast, the present embodiment employs an oxygen-saturation-acquiring unit 15 that is configured to calculate the oxygen saturation from the ratio (R/IR) between the first received-light intensity and the second received-light intensity detected by the light-receiving units 11 and from the particular calibration curve selected in correspondence with the blood concentration.

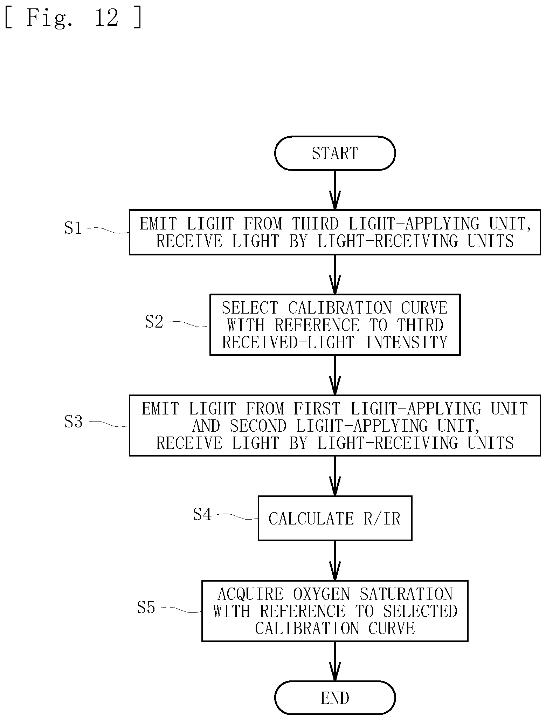

Now, the process of calculating the oxygen saturation according to the present embodiment will be described with reference to the flow chart illustrated in Fig. 12.

A plurality of calibration curves (C1 to C4) corresponding to blood concentrations (hematocrit values) are stored in advance in the calibration-curve-storing unit 13, and the patient's blood is caused to extracorporeally circulate through the blood circuit. Then, the third light-applying units 10 emit the detection light, and the light-receiving units 11 receive the reflection of the detection light to acquire the third received-light intensity (IR_810) (S1). With reference to the third received-light intensity (IR_810) thus acquired, a particular calibration curve that corresponds to the hematocrit value is selected (S2).

Subsequently, the first light-applying unit 8 and the second light-applying unit 9 emit the red light and the near infrared light, and the light-receiving units 11 acquire the first received-light intensity (R_660) and the second received-light intensity (IR_880) (S3). Then, the ratio (R/IR) between the first received-light intensity (R_660) and the second received-light intensity (IR_880) is calculated (S4). Subsequently, with reference to the particular calibration curve corrected in S2, the oxygen-saturation-acquiring unit 15 acquires the oxygen saturation (S5).

According to the present embodiment, the error-absorbing unit 12 includes the calibration-curve-storing unit 13 configured to store in advance a plurality of calibration curves (C1 to C4) that correspond to blood concentrations and that are to be used in obtaining the oxygen saturation of the blood from the ratio (R/IR) between the first received-light intensity (R_660) and the second received-light intensity (IR_880); the calibration-curve-selecting unit 19 configured to select, with reference to the third received-light intensity (IR_810) detected by the light-receiving units 11, a particular one of the plurality of calibration curves (C1 to C4) stored in the calibration-curve-storing unit 13; and the oxygen-saturation-acquiring unit 15 configured to acquire, with reference to the calibration curve selected by the calibration-curve-selecting unit 19, the oxygen saturation of the blood from the ratio (R/IR) between the first received-light intensity (R_660) and the second received-light intensity (IR_880) detected by the light-receiving units 11. Therefore, the oxygen saturation can be acquired with neither the correction of the ratio (R/IR) between the first received-light intensity (R_660) and the second received-light intensity (IR_880) detected by the light-receiving units 11 nor the correction of the calibration curve.

Now, a blood purification apparatus according to a fourth embodiment of the present invention will be described. Elements that are the same as in the above embodiments are denoted by corresponding ones of the reference signs, and detailed description of such elements is omitted.

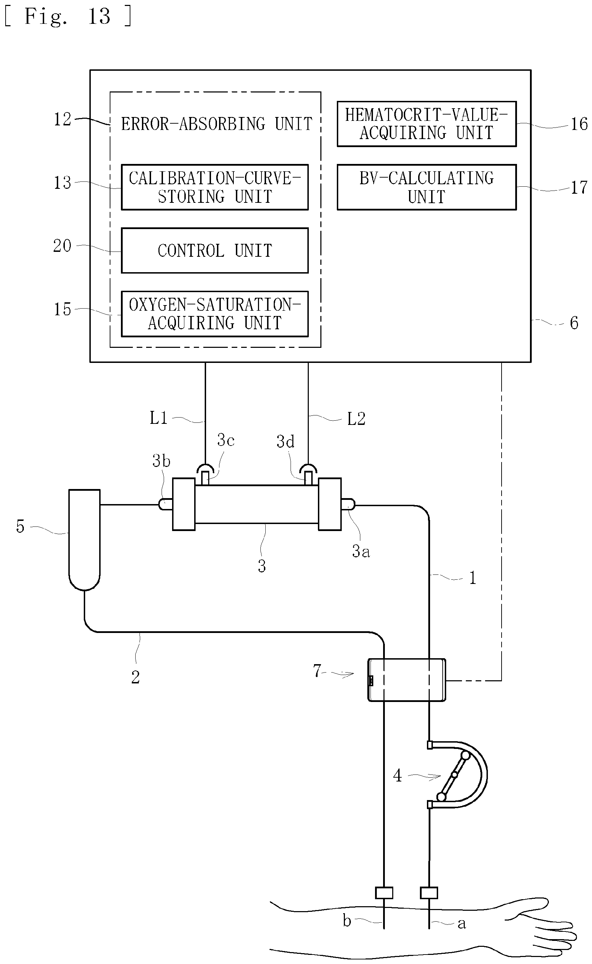

A blood purification apparatus according to the present embodiment is a dialysis apparatus for giving dialysis treatment and includes, as illustrated in Fig. 13, a blood circuit, which includes an arterial blood circuit 1 and a venous blood circuit 2; a dialyzer 3 (a blood purifier), which is interposed between the arterial blood circuit 1 and the venous blood circuit 2 and is configured to purify blood that flows through the blood circuit; a blood pump 4; an air-trap chamber 5, which is provided to the venous blood circuit 2; a dialysis device 6, which is configured to supply dialysate to the dialyzer 3 and discharge drain liquid from the dialyzer 3; and a blood-index detector 7.

The dialysis device 6 according to the present embodiment includes an error-absorbing unit 12, a hematocrit-value-acquiring unit 16, and a BV-calculating unit 17. The error-absorbing unit 12 includes a calibration-curve-storing unit 13, a control unit 20, and an oxygen-saturation-acquiring unit 15. The error-absorbing unit 12 includes, for example, a microcomputer and a storage that are electrically connected to the blood-index detector 7. The error-absorbing unit 12 is configured to acquire oxygen saturation ($SO_2$(ABL)%) from the ratio (R/IR) between the first received-light intensity (R_660) and the second received-light intensity (IR_880) and to absorb any error in the oxygen saturation with reference to the third received-light intensity (IR_810) detected by the light-receiving units 11. The error in the oxygen saturation may occur with a change in the blood concentration (hematocrit value) .

The control unit 20 is configured to control, with reference to the third received-light intensity (IR_810) detected by the light-receiving units 11, the emission intensities (irradiation intensities) for the red light and the near infrared light to be emitted from the first light-applying unit 8 and the second light-applying unit 9. Specifically, when the light absorbance of the blood changes with a change in the blood concentration (hematocrit value), the first received-light intensity (R_660) and the second received-light intensity (IR_880) also change in correlation with the rate of the change, causing an error. To absorb (to cancel out) such an error, the control unit 20 controls, in correspondence with the third received-light intensity (IR_810) that correlates with the blood concentration (hematocrit value), the emission intensities for the red light and the near infrared light to be emitted from the first light-applying unit 8 and the second light-applying unit 9.

The oxygen-saturation-acquiring unit 15 is configured to acquire, with reference to the calibration curve stored in the calibration-curve-storing unit 13, the oxygen saturation of the blood from the ratio (R/IR) between the first received-light intensity (R_660) and the second received-light intensity (IR_880) detected by the light-receiving units 11. To summarize, the first to third embodiments each employ an oxygen-saturation-acquiring unit 15 that is configured to calculate the oxygen saturation while maintaining the emission intensities of the first light-applying unit 8 and the second light-applying unit 9 to be constant. In contrast, the present embodiment employs an oxygen-saturation-acquiring unit 15 that is configured to calculate the oxygen saturation by using the control unit 20 configured to control the emission intensities of the first light-applying unit 8 and the second light-applying unit 9 in correspondence with the third received-light intensity (IR_810) that correlates with the blood concentration (hematocrit value).

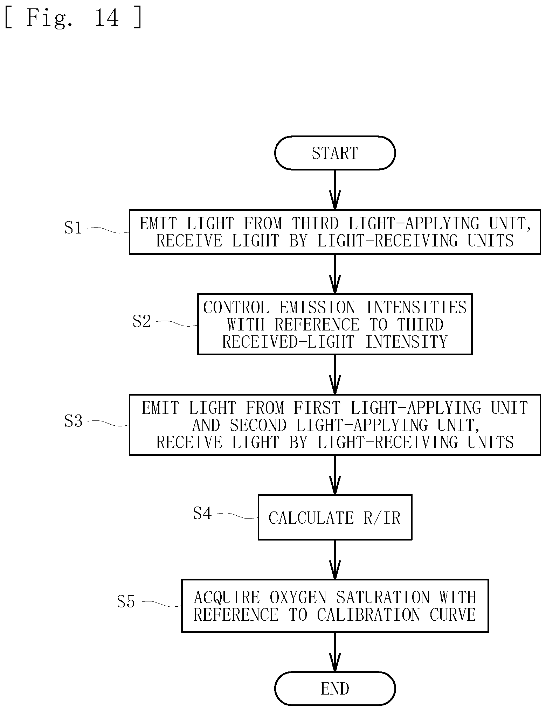

Now, the process of calculating the oxygen saturation according to the present embodiment will be described with reference to the flow chart illustrated in Fig. 14.

A calibration curve is stored in advance in the calibration-curve-storing unit 13, and the patient's blood is caused to extracorporeally circulate through the blood circuit. Then, the third light-applying units 10 emit the detection light, and the light-receiving units 11 receive the reflection of the detection light to acquire the third received-light intensity (IR_810) (S1). With reference to the third received-light intensity (IR_810) thus acquired, the emission intensities for the red light and the near infrared light to be emitted from the first light-applying unit 8 and the second light-applying unit 9 are controlled (S2) .

Subsequently, the first light-applying unit 8 and the second light-applying unit 9 emit the red light and the near infrared light at the emission intensities controlled in S2, and the light-receiving units 11 acquire the first received-light intensity (R_660) and the second received-light intensity (IR_880) (S3). Then, the ratio (R/IR) between the first received-light intensity (R_660) and the second received-light intensity (IR_880) is calculated (S4). Subsequently, with reference to the ratio (R/IR) thus calculated and the calibration curve stored in the calibration-curve-storing unit 13, the oxygen-saturation-acquiring unit 15 acquires the oxygen saturation (S5).

According to the present embodiment, the error-absorbing unit 12 includes the calibration-curve-storing unit 13 configured to store in advance a calibration curve to be used in obtaining the oxygen saturation of the blood from the ratio (R/IR) between the first received-light intensity (R_660) and the second received-light intensity (IR_880); the control unit 20 configured to control, with reference to the third received-light intensity (IR_810) detected by the light-receiving units 11, the emission intensities for the red light and the near infrared light to be emitted from the first light-applying unit 8 and the second light-applying unit 9; and the oxygen-saturation-acquiring unit 15 configured to acquire, with reference to the calibration curve stored in the calibration-curve-storing unit 13, the oxygen saturation of the blood from the ratio (R/IR) between the first received-light intensity (R_660) and the second received-light intensity (IR_880). Therefore, the oxygen saturation can be acquired with neither the correction of the ratio (R/IR) between the first received-light intensity (R_660) and the second received-light intensity (IR_880) detected by the light-receiving units 11 nor the correction of the calibration curve.

In the blood purification apparatus according to each of the first to fourth embodiments, any error in the oxygen saturation that may occur with a change in the blood concentration is absorbed with reference to the third received-light intensity (IR_810) detected by the light-receiving units 11. Such a configuration enables the accurate acquisition of the oxygen saturation regardless of variations in blood concentration. In particular, since the blood concentration (hematocrit value) changes successively during blood purification treatment, the oxygen saturation can be acquired accurately in correspondence with the changing blood concentration and in real time so as to be reflected in the treatment.

The red light to be emitted from the first light-applying unit 8 has a wavelength characterized by an absorbance that is higher for reduced hemoglobin contained in the blood than for oxyhemoglobin contained in the blood. The near infrared light to be emitted from second light-applying unit 9 has a wavelength characterized by an absorbance that is higher for oxyhemoglobin contained in the blood than for reduced hemoglobin contained in the blood. The detection light to be emitted from the third light-applying units 10 has a wavelength characterized by an absorbance that is substantially equal between oxyhemoglobin and reduced hemoglobin contained in the blood. Therefore, the oxygen saturation can be acquired more accurately.

The red light to be emitted from the first light-applying unit 8 has a wavelength of 660 nm. The near infrared light to be emitted from second light-applying unit 9 has a wavelength of 880 nm. The detection light to be emitted from the third light-applying units 10 has a wavelength of 810 nm. These wavelengths are typically used in calculating oxygen saturation. Therefore, general-purpose detectors for oxygen saturation can be employed.

The blood purification apparatus includes the hematocrit-value-acquiring unit 16 configured to acquire the hematocrit value with reference to the third received-light intensity (IR_810) detected by the light-receiving units 11, and the BV-calculating unit 17 configured to calculate the circulating-blood-volume rate of change (ΔBV) with reference to the hematocrit value acquired by the hematocrit-value-acquiring unit 16. Therefore, the oxygen saturation can be acquired by utilizing a hematocrit sensor and a BV meter that are included in a typical blood purification apparatus.

The blood-index detector 7 according to the present embodiment includes the body 7a provided with the first light-applying unit 8, the second light-applying unit 9, the third light-applying units 10, and the light-receiving units 11 and having the fitting grooves (7aa and 7ab) into which portions of the blood circuit are to be fitted; the lid 7b being openable and closable on the body 7a and being capable of nipping, when closed, the portions of the blood circuit that are fitted in the respective fitting grooves (7aa and 7ab); and the light-absorbing parts 7bb provided on the lid 7b and absorbing the light emitted from the first light-applying unit 8, the second light-applying unit 9, and the third light-applying units 10. Therefore, light transmitted through the blood is absorbed by the light-absorbing parts 7bb and is therefore less likely to reach the light-receiving units 11 to cause an error.

As described above, the light-absorbing parts 7bb may be a portion of the lid 7b that has a dark color or that is made of a light-absorbing material. Such a configuration reduces the reflection (disturbance) from the lid 7b. The fitting grooves 7aa and 7ab each extend from one end part of the body 7a to the other end part of the body 7a. The light-receiving units 11 are positioned at the centers of the respective fitting grooves 7aa and 7ab. Therefore, disturbing light from the one end part or the other end part is less likely to reach the light-receiving units 11 to cause an error. The first light-applying unit 8, the second light-applying unit 9, the third light-applying units 10, and the light-receiving units 11 are arrayed on the body 7a. The light-receiving units 11 are positioned between the first light-applying unit 8, the second light-applying unit 9, and the third light-applying units 10. Therefore, disturbing light that may cause an error is less likely to reach the light-receiving units 11. The arrangement of the first light-applying unit 8, the second light-applying unit 9, the third light-applying units 10, and the light-receiving units 11 is not limited to a linear arrangement and may be any other arrangement. The light-receiving units 11 do not necessarily need to be positioned between the first light-applying unit 8, the second light-applying unit 9, and the third light-applying units 10.

While some embodiments have been described above, the present invention is not limited thereto. For example, the red light, the near infrared light, and the detection light to be emitted from the first light-applying unit 8, the second light-applying unit 9, and the third light-applying units 10 may have other wavelengths. The blood concentration to be detected with reference to the third received-light intensity (IR_810) is not limited to the hematocrit value and may be any other kind of blood concentration. The first light-applying unit 8, the second light-applying unit 9, the third light-applying units 10, and the light-receiving units 11 provided to the blood-index detector 7 may be arranged in any way. Some of them may be included in another detector. While the above embodiments are each applied to a dialysis apparatus intended for dialysis treatment, the present invention may also be applied to any other apparatus (such as a blood purification apparatus or a plasma adsorption apparatus intended for hemodiafiltration, hemofiltration, or AFBF) that is capable of purifying a patient's blood while causing the blood to extracorporeally circulate.

Industrial Applicability

The present invention is also applicable to any blood purification apparatus having a different appearance, additional functions, or the like within the spirit of the present invention.

Reference Signs List

1 arterial blood circuit
2 venous blood circuit
3 dialyzer (blood purifier)
4 blood pump
5 air-trap chamber
6 dialysis device
7 blood-index detector
7a body
7aa, 7ab fitting groove
7b lid
7ba protrusion

7bb light-absorbing part
7c locking part
8 first light-applying unit
9 second light-applying unit
10 third light-applying unit
11 light-receiving unit
12 error-absorbing unit
13 calibration-curve-storing unit
14 ratio-correcting unit
15 oxygen-saturation-acquiring unit
16 hematocrit-value-acquiring unit
17 BV-calculating unit
18 calibration-curve-correcting unit
19 calibration-curve-selecting unit
20 control unit
g detection switch
K printed circuit board
R_660 first received-light intensity
IR_880 second received-light intensity
IR_810 third received-light intensity
R/IR ratio
C, C1 to C4 calibration curve

## Claims

1. A blood purification apparatus configured to purify a patient's blood by causing the blood to extracorporeally circulate through a blood purifier and a blood circuit, the apparatus comprising:

   a first light-applying unit configured to apply red light to the blood flowing in the blood circuit;
   a second light-applying unit configured to apply near infrared light to the blood flowing in the blood circuit;
   a third light-applying unit configure to apply detection light to the blood flowing in the blood circuit, the detection light having a different wavelength from the red light and the near infrared light to be emitted from the first light-applying unit and the second light-applying unit, the detection light enabling detection of blood concentration regardless of an oxygen saturation of the blood;
   a light-receiving unit configured to detect a first received-light intensity acquired by receiving the red light emitted from the first light-applying unit and reflected by the blood or transmitted through the blood, a second received-light intensity acquired by receiving the near infrared light emitted from the second light-applying unit and reflected by the blood or transmitted through the blood, and a third received-light intensity acquired by receiving the detection light emitted from the third light-applying unit and reflected by the blood or transmitted through the blood; and
   an error-absorbing unit configured to acquire the oxygen saturation from a ratio between the first received-light intensity and the second received-light intensity and to absorb any error in the oxygen saturation with reference to the third received-light intensity detected by the light-receiving unit, the error in the oxygen saturation occurring with a change in the blood concentration.

2. The blood purification apparatus according to Claim 1,
   wherein the error-absorbing unit includes

   a calibration-curve-storing unit configured to store in advance a calibration curve to be used in obtaining the oxygen saturation of the blood from the ratio between the first received-light intensity and the second received-light intensity;
   a ratio-correcting unit configured to correct, with reference to the third received-light intensity detected by the light-receiving unit, the ratio between the first received-light intensity and the second received-light intensity detected by the light-receiving unit; and
   an oxygen-saturation-acquiring unit configured to acquire, with reference to the calibration curve stored in the calibration-curve-storing unit, the oxygen saturation of the blood from the ratio, corrected by the ratio-correcting unit, between the first received-light intensity and the second received-light intensity.

**3.** The blood purification apparatus according to Claim 1,
wherein the error-absorbing unit includes

a calibration-curve-storing unit configured to store in advance a calibration curve to be used in obtaining the oxygen saturation of the blood from the ratio between the first received-light intensity and the second received-light intensity;
a calibration-curve-correcting unit configured to correct, with reference to the third received-light intensity detected by the light-receiving unit, the calibration curve stored in the calibration-curve-storing unit; and
an oxygen-saturation-acquiring unit configured to acquire, with reference to the calibration curve corrected by the calibration-curve-correcting unit, the oxygen saturation of the blood from the ratio between the first received-light intensity and the second received-light intensity detected by the light-receiving unit.

**4.** The blood purification apparatus according to Claim 1,
wherein the error-absorbing unit includes

a calibration-curve-storing unit configured to store in advance a plurality of calibration curves that correspond to the blood concentrations and that are to be used in obtaining the oxygen saturation of the blood from the ratio between the first received-light intensity and the second received-light intensity;
a calibration-curve-selecting unit configured to select, with reference to the third received-light intensity detected by the light-receiving unit, a particular one of the plurality of calibration curves stored in the calibration-curve-storing unit; and
an oxygen-saturation-acquiring unit configured to acquire, with reference to the calibration curve selected by the calibration-curve-selecting unit, the oxygen saturation of the blood from the ratio between the first received-light intensity and the second received-light intensity detected by the light-receiving unit.

**5.** The blood purification apparatus according to Claim 1,
wherein the error-absorbing unit includes

a calibration-curve-storing unit configured to store in advance a calibration curve to be used in obtaining the oxygen saturation of the blood from the ratio between the first received-light intensity and the second received-light intensity;
a control unit configured to control, with reference to the third received-light intensity detected by the light-receiving unit, emission intensities for the red light and the near infrared light to be emitted from the first light-applying unit and the second light-applying unit; and
an oxygen-saturation-acquiring unit configured to acquire, with reference to the calibration curve stored in the calibration-curve-storing unit, the oxygen saturation of the blood from the ratio between the first received-light intensity and the second received-light intensity.

**6.** The blood purification apparatus according to any of Claims 1 to 5, wherein the red light to be emitted from the first light-applying unit has a wavelength **characterized by** an absorbance that is higher for reduced hemoglobin contained in the blood than for oxyhemoglobin contained in the blood; the near infrared light to be emitted from second light-applying unit has a wavelength **characterized by** an absorbance that is higher for oxyhemoglobin contained in the blood than for reduced hemoglobin contained in the blood; and the detection light to be emitted from the third light-applying unit has a wavelength **characterized by** an absorbance that is substantially equal between oxyhemoglobin and reduced hemoglobin contained in the blood.

**7.** The blood purification apparatus according to Claim 6, wherein the red light to be emitted from the first light-applying unit has a wavelength of 660 nm; the near infrared light to be emitted from second light-applying unit has a wavelength of 880 nm; and the detection light to be emitted from the third light-applying unit has a wavelength of 810 nm.

**8.** The blood purification apparatus according to any of Claims 1 to 7, further comprising a hematocrit-value-acquiring unit configured to acquire a hematocrit value with reference to the third received-light intensity detected by the light-receiving unit.

**9.** The blood purification apparatus according to Claim 8, further comprising a BV-calculating unit configured to calculate a circulating-blood-volume rate of change ($\Delta$BV) with reference to the hematocrit value acquired by the hematocrit-value-acquiring unit.

**10.** The blood purification apparatus according to any of Claims 1 to 9, further comprising:

a body provided with the first light-applying unit, the second light-applying unit, the third light-applying unit, and the light-receiving unit and having a fitting groove into which a portion of the blood circuit is to be fitted;
a lid being openable and closable on the body and being capable of nipping, when closed, the portion of the blood circuit that is fitted in the fitting groove; and
a light-absorbing part provided on the lid and absorbing the light emitted from the first light-applying unit, the second light-applying unit, and the third light-applying unit.

**11.** The blood purification apparatus according to Claim 10, wherein the light-absorbing part is a portion of the lid that has a dark color or that is made of a light-absorbing material.

**12.** The blood purification apparatus according to Claim 10 or 11, wherein the fitting groove extends from one end part of the body to an other end part of the body, and the light-receiving unit is positioned at a center of the fitting groove.

**13.** The blood purification apparatus according to Claim 12, wherein the first light-applying unit, the second light-applying unit, the third light-applying unit, and the light-receiving unit are arrayed on the body; and the light-receiving unit is positioned between the first light-applying unit, the second light-applying unit, and the third light-applying unit.

[ Fig. 1 ]

[ Fig. 2 ]

[ Fig. 3 ]

[ Fig. 4 ]

7c

7b

7bb

7ba

7

7bb

11

7aa

α

7a

K

8    10    10    9

[ Fig. 5 ]

HEMATOCRIT VALUE

[ Fig. 6 ]

R/IR

[ Fig. 7 ]

```
                           ┌─────────────┐
                           │    START    │
                           └──────┬──────┘
                                  │
                                  ▼
        ┌──────────────────────────────────────────────────┐
   S1 ──│ EMIT LIGHT FROM THIRD LIGHT-APPLYING UNIT,        │
        │  RECEIVE LIGHT BY LIGHT-RECEIVING UNITS           │
        └───────────────────────┬──────────────────────────┘
                                │
                                ▼
               ┌──────────────────────────────────┐
          S2 ──│     CALCULATE CORRECTION         │
               │      FACTOR FROM THIRD            │
               │   RECEIVED-LIGHT INTENSITY        │
               └───────────────┬──────────────────┘
                               │
                               ▼
        ┌──────────────────────────────────────────────────┐
   S3 ──│ EMIT LIGHT FROM FIRST LIGHT-APPLYING UNIT         │
        │      AND SECOND LIGHT-APPLYING UNIT,              │
        │   RECEIVE LIGHT BY LIGHT-RECEIVING UNITS          │
        └───────────────────────┬──────────────────────────┘
                                │
                                ▼
                   ┌────────────────────────┐
              S4 ──│    CALCULATE R/IR      │
                   └───────────┬────────────┘
                               │
                               ▼
            ┌──────────────────────────────────────┐
       S5 ──│  CORRECT R/IR WITH REFERENCE         │
            │      TO CORRECTION FACTOR            │
            └──────────────────┬───────────────────┘
                               │
                               ▼
        ┌──────────────────────────────────────────────┐
   S6 ──│ ACQUIRE OXYGEN SATURATION WITH               │
        │ REFERENCE TO CALIBRATION CURVE               │
        └───────────────────────┬──────────────────────┘
                                │
                                ▼
                         ┌─────────────┐
                         │     END     │
                         └─────────────┘
```

[ Fig. 8 ]

[ Fig. 9 ]

```
                      ┌─────────────┐
                      │    START    │
                      └─────────────┘
                             │
                             ▼
      ┌───────────────────────────────────────────────┐
  S1  │ EMIT LIGHT FROM THIRD LIGHT-APPLYING UNIT,     │
      │ RECEIVE LIGHT BY LIGHT-RECEIVING UNITS         │
      └───────────────────────────────────────────────┘
                             │
                             ▼
           ┌─────────────────────────────────┐
  S2       │      CALCULATE CORRECTION        │
           │       FACTOR FROM THIRD          │
           │    RECEIVED-LIGHT INTENSITY      │
           └─────────────────────────────────┘
                             │
                             ▼
         ┌───────────────────────────────────────┐
  S3     │ CORRECT CALIBRATION CURVE WITH         │
         │ REFERENCE TO CORRECTION FACTOR         │
         └───────────────────────────────────────┘
                             │
                             ▼
      ┌───────────────────────────────────────────────┐
  S4  │ EMIT LIGHT FROM FIRST LIGHT-APPLYING UNIT      │
      │ AND SECOND LIGHT-APPLYING UNIT,                │
      │ RECEIVE LIGHT BY LIGHT-RECEIVING UNITS         │
      └───────────────────────────────────────────────┘
                             │
                             ▼
              ┌───────────────────────────┐
  S5          │      CALCULATE R/IR        │
              └───────────────────────────┘
                             │
                             ▼
         ┌───────────────────────────────────────┐
  S6     │   ACQUIRE OXYGEN SATURATION            │
         │      WITH REFERENCE TO                 │
         │ CORRECTED CALIBRATION CURVE            │
         └───────────────────────────────────────┘
                             │
                             ▼
                      ┌─────────────┐
                      │     END     │
                      └─────────────┘
```

[ Fig. 10 ]

[ Fig. 11 ]

[ Fig. 12 ]

```
                    ┌─────────────┐
                    │    START    │
                    └──────┬──────┘
                           ▼
S1 ──  ┌──────────────────────────────────────────────┐
       │  EMIT LIGHT FROM THIRD LIGHT-APPLYING UNIT,   │
       │    RECEIVE LIGHT BY LIGHT-RECEIVING UNITS     │
       └──────────────────────┬───────────────────────┘
                              ▼
S2 ──        ┌──────────────────────────────┐
             │   SELECT CALIBRATION CURVE    │
             │    WITH REFERENCE TO THIRD     │
             │    RECEIVED-LIGHT INTENSITY    │
             └───────────────┬───────────────┘
                             ▼
S3 ──  ┌──────────────────────────────────────────────┐
       │  EMIT LIGHT FROM FIRST LIGHT-APPLYING UNIT    │
       │     AND SECOND LIGHT-APPLYING UNIT,           │
       │    RECEIVE LIGHT BY LIGHT-RECEIVING UNITS     │
       └──────────────────────┬───────────────────────┘
                              ▼
S4 ──            ┌──────────────────────┐
                 │    CALCULATE R/IR     │
                 └──────────┬───────────┘
                            ▼
S5 ──        ┌──────────────────────────────┐
             │  ACQUIRE OXYGEN SATURATION    │
             │    WITH REFERENCE TO SELECTED  │
             │       CALIBRATION CURVE        │
             └───────────────┬───────────────┘
                             ▼
                    ┌─────────────┐
                    │     END     │
                    └─────────────┘
```

[ Fig. 13 ]

[ Fig. 14 ]

```
                        ┌─────────────┐
                        │    START    │
                        └──────┬──────┘
                               │
                               ▼
    ┌──────────────────────────────────────────────────────┐
S1  │    EMIT LIGHT FROM THIRD LIGHT-APPLYING UNIT,         │
    │     RECEIVE LIGHT BY LIGHT-RECEIVING UNITS            │
    └──────────────────────────┬───────────────────────────┘
                               │
                               ▼
          ┌──────────────────────────────────────┐
S2        │    CONTROL EMISSION INTENSITIES       │
          │      WITH REFERENCE TO THIRD          │
          │      RECEIVED-LIGHT INTENSITY         │
          └──────────────────┬───────────────────┘
                             │
                             ▼
    ┌──────────────────────────────────────────────────────┐
S3  │    EMIT LIGHT FROM FIRST LIGHT-APPLYING UNIT          │
    │         AND SECOND LIGHT-APPLYING UNIT,               │
    │     RECEIVE LIGHT BY LIGHT-RECEIVING UNITS            │
    └──────────────────────────┬───────────────────────────┘
                               │
                               ▼
                    ┌────────────────────┐
S4                  │   CALCULATE R/IR   │
                    └─────────┬──────────┘
                              │
                              ▼
          ┌──────────────────────────────────────┐
S5        │   ACQUIRE OXYGEN SATURATION WITH      │
          │   REFERENCE TO CALIBRATION CURVE      │
          └──────────────────┬───────────────────┘
                             │
                             ▼
                      ┌─────────────┐
                      │     END     │
                      └─────────────┘
```

## INTERNATIONAL SEARCH REPORT

International application No.

**PCT/JP2022/037514**

### A. CLASSIFICATION OF SUBJECT MATTER

*A61M 1/16*(2006.01)i; *G01N 21/27*(2006.01)i; *G01N 21/359*(2014.01)i; *A61B 5/1455*(2006.01)i
FI: A61M1/16 111; G01N21/27 F; G01N21/359; A61B5/1455

According to International Patent Classification (IPC) or to both national classification and IPC

### B. FIELDS SEARCHED

Minimum documentation searched (classification system followed by classification symbols)

A61M1/16; G01N21/27; G01N21/359; A61B5/1455

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2016-125 A (NIKKISO CO., LTD.) 07 January 2016 (2016-01-07) paragraphs [0025], [0042]-[0054], fig. 1-10 | 1-13 |
| Y | WO 2007/105805 A1 (KAWASUMI LAB., INC.) 20 September 2007 (2007-09-20) p. 4, line 17 to p. 5, line 3, p. 12, lines 16-27, p. 14, lines 9-22, p. 15, line 21 to p. 16, line 11, fig. 4, 8 | 1-13 |
| Y | JP 64-29738 A (TERUMO CORP.) 31 January 1989 (1989-01-31) p. 3, lower right column, line 3 to p. 4, line 20, fig. 14a-15 | 2-5 |
| A | | 1, 6-13 |
| A | JP 2003-508144 A (OPTOQ AB) 04 March 2003 (2003-03-04) entire text, all drawings | 1-13 |
| A | WO 2019/180068 A1 (GAMBRO LUNDIA AB) 26 September 2019 (2019-09-26) entire text, all drawings | 1-13 |
| A | US 6360113 B1 (DATEX-OHMEDA, INC.) 19 March 2002 (2002-03-19) entire text, all drawings | 1-13 |

☐ Further documents are listed in the continuation of Box C.　　☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **24 November 2022** | **06 December 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**
Information on patent family members

International application No.

**PCT/JP2022/037514**

| Patent document cited in search report | | | Publication date (day/month/year) | Patent family member(s) | | | Publication date (day/month/year) |
|---|---|---|---|---|---|---|---|
| JP | 2016-125 | A | 07 January 2016 | (Family: none) | | | |
| WO | 2007/105805 | A1 | 20 September 2007 | (Family: none) | | | |
| JP | 64-29738 | A | 31 January 1989 | US | 5149503 | A | |
| | | | | column 1, line 15 to column 2, line 29, fig. 14a-15 | | | |
| | | | | WO | 1989/001144 | A1 | |
| | | | | EP | 380664 | A1 | |
| JP | 2003-508144 | A | 04 March 2003 | WO | 2001/017421 | A1 | |
| | | | | entire text, all drawings | | | |
| WO | 2019/180068 | A1 | 26 September 2019 | US | 2021/0015990 | A1 | |
| | | | | entire text, all drawings | | | |
| | | | | EP | 3542707 | A1 | |
| | | | | KR | 10-2020-0135821 | A | |
| | | | | CN | 112203578 | A | |
| US | 6360113 | B1 | 19 March 2002 | WO | 2001/043632 | A1 | |
| | | | | entire text, all drawings | | | |

Form PCT/ISA/210 (patent family annex) (January 2015)

**REFERENCES CITED IN THE DESCRIPTION**

**Patent documents cited in the description**

- JP 2012040058 A